# EUROPEAN PATENT SPECIFICATION

(11) **EP 3 795 700 B1**
(45) Date of publication and mention of the grant of the patent: **06.12.2023**
(21) Application number: 19198343.6
(22) Date of filing: 19.09.2019
(51) Int. Cl.: C12Q 1/6883

(54) **METHOD FOR IDENTIFYING AN EARLY STAGE OF A CARDIOMYOPATHY**
VERFAHREN ZUR IDENTIFIZIERUNG EINES FRÜHEN STADIUMS EINER KARDIOMYOPATHIE
PROCÉDÉ D'IDENTIFICATION D'UN STADE PRÉCOCE D'UNE CARDIOMYOPATHIE

(43) Date of publication of application: 24.03.2021
(73) Proprietor: IKDT Institut Kardiale Diagnostik und Therapie GmbH, 12203 Berlin (DE)
(72) Inventor: Schultheiss, Hans-Peter, 14163 Berlin (DE); Aleshcheva, Ganna, 10557 Berlin (DE)
(74) Representative: Maikowski & Ninnemann Patentanwälte Partnerschaft mbB

(56) References cited:
- Anonymous: "TPA: Homo sapiens microRNA hsa-miR-21-5p", GenBank, 3 March 2015 (2015-03-03), XP055662792, Retrieved from the Internet: URL:https://www.ncbi.nlm.nih.gov/nuccore/L M378763.1 [retrieved on 2020-01-28]
- Anonymous: "TPA: Homo sapiens microRNA hsa-miR-30a-5p", GenBank, 3 March 2015 (2015-03-03), XP055662795, Retrieved from the Internet: URL:https://www.ncbi.nlm.nih.gov/nuccore/L M378774.1 [retrieved on 2020-01-28]
- PAWEL RUBIS ET AL: "Relations between circulating microRNAs (miR-21, miR-26, miR-29, miR-30 and miR-133a), extracellular matrix fibrosis and serum markers of fibrosis in dilated cardiomyopathy", INTERNATIONAL JOURNAL OF CARDIOLOGY, vol. 231, 1 March 2017 (2017-03-01), pages 201-206, XP055662580, AMSTERDAM, NL ISSN: 0167-5273, DOI: 10.1016/j.ijcard.2016.11.279
- MAMORU SATOH ET AL: "A Cellular MicroRNA,, Is a Novel Biomarker for Clinical Outcome in Patients With Dilated Cardiomyopathy", JOURNAL OF CARDIAL FAILURE, CHURCHILL LIVINGSTONE, NAPERVILLE, IL, US, vol. 17, no. 11, 28 July 2011 (2011-07-28) , pages 923-929, XP028332340, ISSN: 1071-9164, DOI: 10.1016/J.CARDFAIL.2011.07.012 [retrieved on 2011-08-04]
- HONG-FEI XU ET AL: "MicroRNA-21 regulation of the progression of viral myocarditis to dilated cardiomyopathy", MOLECULAR MEDICINE REPORTS, vol. 10, no. 1, 1 July 2014 (2014-07-01), pages 161-168, XP055662756, GR ISSN: 1791-2997, DOI: 10.3892/mmr.2014.2205
- YARON GOREN ET AL: "Serum levels of microRNAs in patients with heart failure", EUROPEAN JOURNAL OF HEART FAILURE, vol. 14, no. 2, 1 February 2012 (2012-02-01), pages 147-154, XP055137648, ISSN: 1388-9842, DOI: 10.1093/eurjhf/hfr155

## Description

The present invention relates to an in-vitro method for identifying an early stage of a cardiomyopathy independent of a hemodynamic impairment of a patient's heart according to claim 1 and to the use of at least one microRNA selected from a specific group as marker in such an in-vitro method according to claim 5.

Endomyocardial biopsies (EMBs) are the gold standard for identification of causative factors in patients with unexplained heart failure as a prerequisite of an aetiology-driven treatment. Nevertheless, EMB is used infrequently because of missing non-invasive, clear defined diagnostic criteria.

MicroRNAs (miRNA) are small, 19 to 23 nucleotides long non-coding RNAs that bind to complementary sequences on the 3' untranslated region of target messenger RNAs (mRNA) post-transcriptionally regulating mRNA expression. They are essential in numerous molecular regulatory pathways involved in most biological processes and have been shown to be unique to the source material (i.e. serum, plasma, tissue) and the disease process being investigated. It is already known that miRNAs are involved in cardiac differentiation, proliferation, cell maturation, and apoptosis as well as in myocardial injury, inflammation, cardiac remodeling, and fibrosis. Because of their stability in the circulation, miRNAs, typically in the form of miRNA profiles, have emerged as prospective biomarkers for many human diseases, in particular cancer or cardiovascular diseases, providing novel molecular insight and new therapeutic strategies to treat diseases.

In fact, circulating miRNAs are shielded from RNA-degrading enzymes, because they are found within vesicles, such as microparticles, exosomes, and apoptotic bodies making them excellent candidate biomarkers for various diseases, including myocardial infarction [1] and heart failure [2, 3] Cardio-enriched miRNAs play a crucial role in cardiac development [4, 5, 6] and have been associated with the development of dilated cardiomyopathy (DCM) [7, 8]. Despite their promise, miRNAs still have not entered the clinical scenario, mainly because of a lack of large cohort studies, or several challenges related to technical aspects, miRNAs normalization, drugs interaction, and quality reporting of statistical multivariable analysis of the miRNAs observational studies [9].

GenBank accession number LM378763 describes the microRNA hsa-miR-21-5p.

GenBank accession number LM378774 describes the microRNA hsa-miR-30a-5p.

Rubiś et al. (Rubiś, Pawe 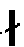 , et al. "Relations between circulating microRNAs (miR-21, miR-26, miR-29, miR-30 and miR-133a), extracellular matrix fibrosis and serum markers of fibrosis in dilated cardiomyopathy." International Journal of Cardiology 231 (2017): 201-206.) describes that microRNAs control a variety of cellular processes essential to the heart, wherein five microRNAs, namely, miR-21, miR-26, miR-29, miR-30, and miR-133awere found to be linked to ECM fibrosis.

Satoh et al. (Satoh, Mamoru, et al. "A cellular microRNA, let-7i, is a novel biomarker for clinical outcome in patients with dilated cardiomyopathy." Journal of Cardiac Failure 17.11 (2011): 923-929.) addresses the question whether the microRNAs *let-7i,* miR-21, miR-126, and miR-155 are expressed with TLR4 in human dilated cardiomyopathy (DCM), and whether *let-7i* levels are related to clinical outcomes. It was found that a decrease in *let-7i* may be related to poor clinical outcomes in patients with DCM.

Xu et al. (Xu, Hong-Fei, et al. "MicroRNA-21 regulation of the progression of viral myocarditis to dilated cardiomyopathy." Molecular Medicine Reports 10.1 (2014): 161-168.) investigated miR-21 expression and its potential role in viral myocarditis (VMC) and DCM. The described results suggest a regulatory role for miR-21. The findings revealed that changes in the expression of miRNAs may contribute to the pathogenesis of VMC to DCM and establish the therapeutic efficacy of miRNA targeted intervention in a cardiovascular disease setting.

Goren et al. (Goren, Yaron, et al. "Serum levels of microRNAs in patients with heart failure." European Journal of Heart Failure 14.2 (2012): 147-154.) found that elevated serum levels of specific microRNAs, namely, miR-423-5p, miR-320a, miR-22, and miR-92b, identify systolic heart failure patients and correlate with important clinical prognostic parameters.

It is an object of the present invention to indicate novel applications of microRNAs in the field of identifying and classifying cardiomyopathies.

This object is achieved by an in-vitro method for identifying an early stage of the cardiomyopathy having the features of claim 1.

Such an in-vitro method is suited to identify an early stage of a cardiomyopathy independent on the hemodynamic impairment of a patient's heart. Thus, even of no clinical symptoms can be detected, i.e., if the patient's heart clinically appears to be healthy, the presently claimed method is able to identify an early stage of a cardiomyopathy. This is done by analyzing a blood serum/blood plasma sample of a patient to determine in a quantitative or semiquantitative manner an expression level of at least one microRNA selected from the group consisting of the following microRNAs: hsa-miR-21-5p (SEQ ID NO: 1) and hsa-miR-30a-5p (SEQ ID NO: 2).

It turned out that either of these two microRNAs is particularly appropriate to identify an early stage of a cardiomyopathy independent on a hemodynamic impairment of the patient's heart. Thus, prior to an onset of clinical symptoms, the expression level of either of these two microRNAs can be used as indication of an early stage of a cardiomyopathy, in particular of an early stage of a dilated cardiomyopathy (DCM).

An expression level of the at least one microRNA being higher than in a healthy control group and/or in a group of patients suffering from an inflammatory myocardial disease and/or a group of patients suffering from a virally induced cardiomyopathy is taken as an indicator for the presence of an early stage of a cardiomyopathy. Thus, it is possible by simply identifying the expression level of the at least one microRNA to distinguish an early stage of a cardiomyopathy both from a healthy donor of the blood serum/blood plasma sample and from patients suffering from an inflammatory myocardial disease and/or suffering from a virally-induced cardiomyopathy. Therewith, the two microRNAs can be individually seen as "super marker" for distinguishing patients suffering from an early stage of a cardiomyopathy from other myocardial patient as well as from healthy individuals. It was a completely surprising finding that the expression level of the at least one microRNA is increased in case of an early stage of a cardiomyopathy with respect to both patients having an advanced myocardial disease (in particular including an inflammation of the myocardium and/or a virally-induced cardiomyopathy) and healthy individuals.

The early stage of the cardiomyopathy is characterized by the absence of a viral infection of the patient's heart. Additionally or alternatively, the early stage of the cardiomyopathy is characterized by an absence of an inflammation of the patient's heart. Thus, even if no typical disease-related symptoms or conditions are present within the patient's heart, the presently claimed in-vitro method can be used to identify an early stage of a cardiomyopathy.

In an embodiment, the claimed in-vitro methods can be used to identify patients that require an endomyocardial biopsy (EMB) to verify the finding that the patient suffers from an early stage of a cardiomyopathy.

In an embodiment, the hemodynamic parameter is the ejection fraction of the patient. Healthy individuals typically have ejection fractions between 55 % and 65 %. Often, an ejection fraction of 55 % is used to distinguish healthy individuals from diseased patients. In an embodiment, the presently claimed method can be applied both to individuals having in ejection fraction of more than 55 % and to individuals having an ejection fraction of equal to or less than 55 %.

In an embodiment, the patient has no clinically noticeable cardiac insufficiency. Thus, the patient is, in this embodiment, clinically indistinguishable from healthy control persons. Nonetheless, an analysis with the presently claimed in-vitro method reveals an early stage of a cardiomyopathy of the patient so that early counteractions can be started, such as an appropriate therapy of the cardiomyopathy prior to onset of clinically noticeable symptoms.

To further increase the clinically informative value of the presently claimed method, the expression level of at least one additional microRNA is determined in a semiquantitative or quantitative manner in an embodiment. Thereby, the at least one additional microRNA is chosen from the group consisting of: hsa-let-7f-5p (SEQ ID NO: 3), hsa-miR-197-3p (SEQ ID NO: 4), hsa-miR-223-3p (SEQ ID NO: 5), hsa-miR-379-5p (SEQ ID NO: 6), and hsa-miR-93-5p (SEQ ID NO: 7).

It was found by the inventors that the expression levels of any of these additional microRNAs is altered in case of myocardial diseases. Thereby, hsa-let-7f-5p (SEQ ID NO: 3) and hsa-miR-223-3p (SEQ ID NO: 5) are typically individually or concomitantly down-regulated with respect to healthy donors in case of patients suffering from a myocardial disease. Thus, a down-regulation of either of these microRNAs can be seen as additional indicator for the presence of an early stage of a cardiomyopathy. However, down-regulation of either of these additional microRNAs would not be sufficient to distinguish an early stage of a cardiomyopathy from other cardiomyopathies (in particular those involving a viral infection and/or inflammation of the myocardium).

Likewise, an up-regulation of either of the additional microRNAs hsa-miR-197-3p (SEQ ID NO: 4), hsa-miR-379-5p (SEQ ID NO: 6), and hsa-miR-93-5p (SEQ ID NO: 7) can be seen as additional indicator that an early stage of a cardiomyopathy is present since these microRNAs are individually or concomitantly typically upregulated with respect to healthy donors in case of patients suffering from a cardiomyopathy. Once again, an up-regulation of the expression levels of either of these additional microRNAs is not yet sufficient to distinguish an early stage of the cardiomyopathy from other cardiomyopathies involving a viral infection and/or an inflammation and later stage of the disease.

In an embodiment, the in-vitro methods comprises the steps explained the following.

First, a sample obtained from an apparently healthy individual or a patient suspected of suffering from a cardiomyopathy is provided.

Afterwards, this sample is brought into contact with at least one probe comprising a sequence corresponding to any of the sequences of hsa-miR-21-5p (SEQ ID NO: 1) and hsa-miR-30a-5p (SEQ ID NO: 2) and optionally to any of the sequences of hsa-let-7f-5p (SEQ ID NO: 3), hsa-miR-197-3p (SEQ ID NO: 4), hsa-miR-223-3p (SEQ ID NO: 5), hsa-miR-379-5p (SEQ ID NO: 6), and hsa-miR-93-5p (SEQ ID NO: 7). Alternatively, the at least one probe might have a sequence being complementary to the sequences of the precedingly listed microRNAs. The bringing into contact is performed under conditions allowing hybridization between microRNAs contained in the sample and the at least one probe.

Afterwards, a hybridization or hybridization level between microRNAs in the sample and the at least one probe is determined in a semiquantitative or quantitative manner.

Finally, a relative or absolute expression level of a microRNA in the sample is confirmed using the hybridization result of the previous step.

These method steps particularly easy allow the determination of a relative absolute expression level of the microRNAs of interest.

In an embodiment, the determined expression level of the at least one microRNA is normalized with respect to the expression level of hsa-miR-30a-3p (SEQ ID NO: 8) in the same sample.

In an embodiment, a threshold of the relative expression of hsa-miR-21-5p (SEQ ID NO: 1) with respect to hsa-miR-30a-3p (SEQ ID NO: 8) lying in a range between 10 and 20, in particular between 11 and 19, in particular between 12 and 18, in particular between 13 and 17, in particular between 14 and 16 is applied to distinguish between patients experiencing an early stage of a cardiomyopathy and patients not experiencing such an early stage of a cardiomyopathy. Thereby, the presence of an early stage of a cardiomyopathy is assumed if the relative expression of hsa-miR-21-5p (SEQ ID NO: 1) lies above the threshold.

In an embodiment, a threshold of the relative expression of hsa-miR-30a-5p (SEQ ID NO: 2) with respect to hsa-miR-30a-3p (SEQ ID NO: 8) lying in a range between 5 and 10, in particular between 6 and 9, in particular between 7 and 8 is applied to distinguish between patients experiencing an early stage of a cardiomyopathy and patients not experiencing such an early stage of a cardiomyopathy. Thereby, the presence of an early stage of a cardiomyopathy is assumed if the relative expression of hsa-miR-30a-5p (SEQ ID NO: 2) lies above the threshold. In an aspect, the present invention relates to the in-vitro use of at least one microRNA selected from the group consisting of hsa-miR-21-5p (SEQ ID NO: 1) and hsa-miR-30a-5p (SEQ ID NO: 2) as marker in an in-vitro method for identifying an early stage of a cardiomyopathy independent on a hemodynamic impairment of the patient's heart. Thereby, an expression level of the at least one microRNA is determined in a blood serum/blood plasma sample of the patient in a semiquantitative quantitative manner when applying the novel use of the at least one microRNA.

Herewith disclosed but not part of the invention is the further medical use of at least one microRNA selected from the group consisting of hsa-miR-21-5p (SEQ ID NO: 1) and hsa-miR-30a-5p (SEQ ID NO: 2) in in-vivo diagnostics of an early stage of a cardiomyopathy independent on a hemodynamic impairment of the patient's heart.

In an embodiment, an expression level of the at least one microRNA is determined in the blood of the patient in a semiquantitative or quantitative manner within the framework of this novel medical use of the at least one microRNA.

Herewith disclosed is a medical method for diagnosing an early stage of a cardiomyopathy in a patient in need thereof. This medical method can be applied as in-vitro method or as in-vivo method. In vivo methods are not part of the invention.
It comprises the steps explained in the following:
First, a sample obtained from an apparently healthy individual or a patient suspected of suffering from a cardiomyopathy is provided. Alternatively, the method is carried out directly on a patient by using the patient's blood.

Afterwards, the provided sample or the patient's blood is brought into contact with at least one probe comprising a sequence corresponding to any of the sequences of hsa-miR-21-5p (SEQ ID NO: 1) and hsa-miR-30a-5p (SEQ ID NO: 2) and optionally to any of the sequences of hsa-let-7f-5p (SEQ ID NO: 3), hsa-miR-197-3p (SEQ ID NO: 4), hsa-miR-223-3p (SEQ ID NO: 5), hsa-miR-379-5p (SEQ ID NO: 6), and hsa-miR-93-5p (SEQ ID NO: 7). Alternatively, the at least one probe might have a sequence being complementary to the sequences of the precedingly listed microRNAs. The bringing into contact is performed under conditions allowing hybridization between microRNAs contained in the sample or blood on the one hand and the at least one probe on the other hand.

Afterwards, a hybridization or hybridization level between microRNAs in the sample or blood and the at least one probe is determined in a semiquantitative or quantitative manner.

Finally, a relative or absolute expression level of a microRNA in the sample or patient's blood is confirmed using the hybridization result of the previous step.

All embodiments of the described methods can be combined in any desired way and can be transferred from one method to another method. Furthermore, all embodiments of the described methods can be transferred to the described medical or non-medical uses of microRNAs in any desired combination. Likewise, all embodiments of the described uses of the microRNAs can combined in any desired way and can be transferred in an analogous way to the described methods.

Further details of aspects of the present invention will be explained in the following with respect to exemplary embodiments and accompanying Figures. In the Figures:
- Figure 1: shows a volcano plot of microRNA expression levels in patients with inflammatory and/or virally induced myocardial disease versus healthy donors;
- Figure 2A: shows a volcano plot of microRNA expression levels in patients with DCM versus healthy donors;
- Figure 2B: shows a volcano plot of microRNA expression levels in patients with DCM versus patients with inflammatory and/or virally induced myocardial disease;
- Figure 3: shows a confirmation of differentially expressed miRNAs in patients with inflammatory myocardial disease (IMD), DCM, and healthy subjects using qRT-PCR;
- Figure 4: shows the diagnostic value of miRNAs for DCM and unexplained heart failure presented in receiver-operating characteristic (ROC) curves with calculated area under the curve (AUC); and
- Figure 5: shows the influence of the ejection fraction (EF) on the miRNA expression.

The Figures will be explained in the framework of exemplary embodiments in the following.

In the following sections, the term "dilated cardiomyopathy" or "DCM" is used for describing an early stage of a cardiomyopathy that is typically not yet referred to as DCM since no clinically significant symptoms could be observed throughout this patient group. In particular, the patients referred to as DCM patients showed no inflammation of the myocardium nor a virally-induced myocardial disease. Rather, the patients referred to as DCM patients in the following only showed the (non-clinical) symptoms of an early stage of a cardiomyopathy. Thus, the term "DCM" was mainly used for applying a medically accepted term to those patients suffering from an early stage of a cardiomyopathy and to distinguish those patients from patients suffering from a virally-induced cardiomyopathy and/or an inflammation of the myocardium.

### Methods

### Study Patients

The exemplary embodiment composed of two parts (screening and validation). Within the exemplary embodiment, EMB specimens of 414 patients in total with clinically unexplained heart failure (343 patients with inflammatory and/or virally induces heart diseases and 71 patients with DCM) were evaluated based on different disease entities sent to the FDA-validated laboratory IKDT (Institute for Cardiac Diagnostic and Therapy Berlin, Germany). Some of these patients complained about symptoms of heart failure with fatigue, reduced physical capacity or dyspnea on exertion, and cardiac dysfunction. Patients with coronary artery disease, other possible causes of myocardial dysfunction (e.g. valvular heart disease, hypertension, restrictive, or constrictive heart disease) diagnosed by angiography and echocardiography, and concomitant chronic inflammatory disease (e.g. rheumatological disorders) were excluded from this study.

Left ventricular ejection fraction (LVEF) was determined by echocardiography [10]. Therapy at the time of serum samples taking was not known as only blood or serum samples and EMBs were received for diagnostic evaluation.

### Endomyocardial Biopsy analysis

### Histological and Immunohistochemical Staining for Assessment of Inflammation

The patients were grouped based on EMB as followed:
1) Patients having an active myocarditis according to the Dallas criteria [11] as a histological evidence of inflammatory infiltrates within the myocardium associated with myocyte degeneration and necrosis.
2) Patients having a borderline myocarditis (LVEF > 45 %) with histological evidence of inflammatory infiltrates of >14.0 lymphocytes/mm², including >7.0 CD3+ lymphocytes/mm² according to the European Society of Cardiology guidelines within the myocardium without myocyte degeneration and necrosis.
3) Patients having inflammatory cardiomyopathy (DCMi) (LVEF < 45 %) in the absence of viral genomes by evidence of intramyocardial inflammation with immunohistological evidence of >14.0 lymphocytes/mm², including >7.0 CD3⁺ lymphocytes/mm² according to the European Society of Cardiology guidelines [12]. Furthermore, macrophages were analyzed (threshold >40.0 CD11b⁺/Mac-1⁺ macrophages/mm²), LFA-1⁺, CD45RO, and the expression of HLA-1 (cf. Table 2) [13, 14].
4) Patients having idiopathic giant cell myocarditis and displaying a rather diffuse infiltration of cardiac tissue with a mixture of inflammatory cells, e.g. lymphocytes, macrophages, multinucleated giant cells and/or disseminated necrosis of myocytes. The time from symptoms onset and EMB was not known.
5) Patients having a dilated cardiomyopathy (DCM) was made on morphological and functional characterization with significantly impaired LVEF (LVEF < 45 %) and/or dilated LV (Table 1). EMBs from DCM patients were negative for histologically- or immunohistologically-detected inflammation, and for the detection of cardiotropic viral genomes.

Patients with a genetic form of cardiomyopathy were excluded from the study.

Used antibodies and immunohistological staining procedure and evaluation of EMBs can be found elsewhere [15, 16].

**Table 1: Clinical and hemodynamic data of all patients.**

| **Characteristic** | **Inflammatory myocardial disease patients** | **DCM patients** | **Healthy controls** | **All Patients** |
|---|---|---|---|---|
| **Number** | **343** | **71** | **85** | **499** |
| **Age, median (range), y** | **47 (20-78)** | **57 (44-66)** | **39 (28-61)** | **48 (28-78)** |
| **Male sex (%)** | **268 (78)** | **38 (53)** | **27 (31)** | **333 (67)** |
| **LVEF, median (range), %** | **52 (10-70)** | **30 (15-43)** | **-** | **53 (10-70)** |
| **LVEDD, mm** | **55.7 (38.0-76.0)** | **66.3 (44.0-82.0)** | **-** | **58.3 (38.0 - 82.0)** |

### Detection of Viral Genomes

DNA and RNA were extracted from frozen heart muscle tissue probes. RT-PCR (reverse transcriptase polymerase chain reaction) was performed for the detection of enteroviruses (including Coxsackievirus), adenovirus, parvovirus B19, and human herpesvirus type 6 (cf. Table 2) using methods published previously [17, 18]. In addition, DNA was extracted from peripheral blood cells to exclude a systemic infection with PVB19, and HHV6. As a control for successful extraction of DNA and RNA from heart muscle tissue, oligonucleotide sequences were chosen from the DNA sequence of the GAPDH gene.

The clinical and EMB-based molecular virological and immunohistochemical data of all study patients are summarized in Table 1 and Table 2.

**Table 2: Immunohistological analysis of EMB (median) of all patients with unexplained heart failure. The calculated objects were related to the unit Heart Area (mm²) or % Area Fraction.**

| | **Diagnosis** | **CD3+ cells /mm²** | **CD45RO+ cells /mm²** | **LFA-1+ cells /mm²** | **Mac-1+ cells /mm²** | **HLA-1 % Area fraction** |
|---|---|---|---|---|---|---|
| **Inflammatory** myocardial disease patients | Active myocarditis (MCA) | 58.78 | 104.18 | 138.27 | 219.23 | 9.43 |
| | Borderline myocarditis | 20.65 | 73.92 | 29.92 | 48.09 | 10.59 |
| | DCM with inflammation (DCMi) | 16.08 | 44.18 | 39.08 | 53.50 | 7.40 |
| | Idiopathic giant cell myocarditis | 27.05 | 147.00 | 192.53 | 183.45 | 12.93 |
| **Virally and/or inflammatory** myocardial disease patients | Adenovirus (ADV) | 0.00 | 0.00 | - | - | 8.05 |
| | Enterovirus (Coxsackie virus) | 2.12 | 16.19 | 11.04 | 20.82 | 6.83 |
| | Human herpes virus 6 (HHV 6) | 2.80 | 19.13 | 8.65 | 25.35 | 6.72 |
| | Parvovirus B19 | 3.86 | 29.31 | 10.15 | 23.69 | 6.80 |
| | Dilated cardiomyopathy (DCM) | 2.85 | 10.69 | 7.85 | 18.05 | 5.37 |

### MiRNAs isolation

MiRNAs were obtained from patients' serum using mirVANA^{™} PARIS^{™} RNA and Native Protein Purification Kit (Invitrogen; Thermo Fisher Scientific, Inc., Waltham, MA, USA) according to manufacturer's instructions.

### MiRNA reverse transcription, pre-amplification and expression analysis using TaqMan^{®} Open Array and qRT-PCR

Total RNA including miRNA fraction was initially reversely transcribed to cDNA using Megaplex stem-loop RT primer (Thermo Fisher Scientific, Waltham, MA, USA) for Human Pool A and B in combination with the TaqMan MicroRNA Reverse Transcription Kit (Thermo Fisher Scientific, Waltham, MA, USA) for low content samples. The entire procedure for quantification of miRNAs is described elsewhere.(54) Hsa-miR-30a-3p was used as reference miRNA used for data normalization.

### Screening of samples for miRNA profile identification

MiRNAs from serum samples of 184 patients with biopsy-proven inflammatory and/or virally-induced heart muscle diseases, 25 patients with dilated cardiomyopathy (DCM), and 25 healthy subjects (in.vent Diagnostica GmbH, Hennigsdorf) were measured using TaqMan^{®} OpenArray^{®} (Table 3). This enabled to measure the expression of 754 unique circulating miRNAs (Human MicroRNA Panels A and B) in each sample to find out the miRNAs of interest (e.g. miRNA profile).

**Table 3: Number of screened samples for miRNA analysis sorted by diagnosis.**

| **Diagnosis by endomyocardial biopsy** | | **Number of serum samples** | | | | |
|---|---|---|---|---|---|---|
| | | **with inflammation** | **without inflammation** | **with virus** | **without virus** | **Total** |
| **Inflammatory** myocardial disease patients | Myocarditis | 29 | 0 | 5 | 24 | 29 |
| | DCM with inflammation (DCMi) | 22 | 0 | 0 | 22 | 22 |
| | Idiopathic giant cell myocarditis | 8 | 0 | 4 | 4 | 8 |
| **Virally and/or inflammatory** myocardial disease patients | *Adenovirus (ADV)* | 2 | 7 | 9 | 0 | 9 |
| | *Enterovirus (coxsackie virus)* | 21 | 60 | 81 | 0 | 81 |
| | *Human herpes virus 6 (HHV6)* | 8 | 4 | 12 | 0 | 12 |
| | *Parvovirus B19 (PVB)* | 10 | 13 | 23 | 0 | 23 |
| **Total** | | | | | | 184 |
| **Dilated cardiomyopathy (DCM) patients** | | 0 | 25 | 0 | 25 | 25 |
| **Healthy blood donors** | | 0 | 25 | 0 | 25 | 25 |

### Validation experiments

After identification of miRNA profile consisting of eight miRNAs with TaqMan^{®} OpenArray^{®}, the results have been confirmed in single assays of qRT-PCR in serum **of another** 159 patients with biopsy-proven inflammatory and/or virally-induced diseases, 46 DCM patients, and 60 healthy donors (Table 4).

**Table 4: Number of validated samples for miRNA analysis sorted by diagnosis.**

| **Diagnosis by endomyocardial biopsy** | | **Number of serum samples** | | | | |
|---|---|---|---|---|---|---|
| | | **with inflammation** | **without inflammation** | **with virus** | **without virus** | **Total** |
| **Inflammatory** myocardial disease patients | Myocarditis | 35 | 0 | 0 | 35 | 35 |
| | DCM with inflammation (DCMi) | 32 | 0 | 0 | 32 | 32 |
| | Idiopathic giant cell myocarditis | 8 | 7 | 0 | 15 | 15 |
| | Virally-induced myocarditis: | | | | | |
| **Virally and/or inflammatory** myocardial disease patients | *Adenovirus (ADV)* | 2 | 16 | 18 | 0 | 18 |
| | *Enterovirus (coxsackie virus)* | 3 | 21 | 24 | 0 | 24 |
| | *Human herpes virus 6 (HHV6)* | 2 | 11 | 13 | 0 | 13 |
| | *Parvovirus B19* | 2 | 20 | 22 | 0 | 22 |
| **Total** | | | | | | **159** |
| **Dilated cardiomyopathy (DCM) patients** | | 0 | 46 | 0 | 46 | 46 |
| **Healthy blood donors** | | 0 | 60 | 0 | 60 | 60 |

### Ethical Approval

The study was performed within the CRC Transregio 19 and was approved by the local ethics committees of the participating clinical centers as well as by the committees of the respective federal states. The study complies with the Declaration of Helsinki. An informed written consent was obtained from each study patient.

### Statistical analysis

Raw data were normalized and analyzed with GraphPad Prism 7 (GraphPad Software, La Jolla, USA). The Student-t-Test and ANOVA were used to compare miRNA expression levels between all study groups.

The receiver-operating characteristic (ROC) curves were plotted for every single miRNA and the areas under the curves (AUCs) were calculated to prove their value and diagnostic accuracy.

All data were presented as single values with mean, with a significance level of * *P* < 0.05, ** *P* <0.005, *** *P* <0.0005, **** *P* <0.0001.

### Results

Serum samples were obtained from patients with biopsy proven inflammatory and/or virally-induced cardiomyopathies (*n* = 343), dilated cardiomyopathy in particular (*n* = 71), and corresponding healthy controls (in.vent Diagnostica GmbH) (*n* = 85) (Table 1, Table 2). All data were generated in the same laboratory to facilitate comparative data analysis.

### Identification of miRNAs differentially expressed in patients with unexplained heart failure using TaqMan^{®} OpenArray^{®} analysis

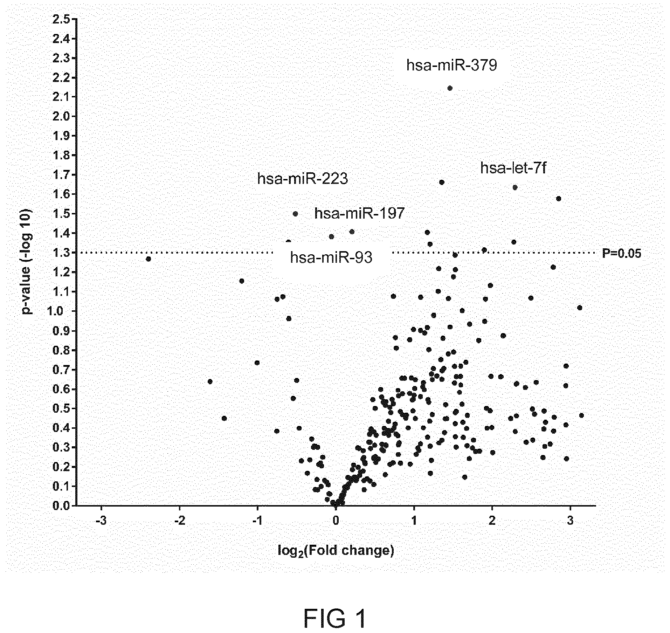

First, miRNA expression studies (screening) were performed with TaqMan^{®} OpenArray^{®} (Thermo Fisher Scientific, Waltham, MA, USA) (Table 3). Based on the expression levels of 323 differently expressed miRNAs related to miR-30a-3p (reference miRNA), using Volcano blot, five miRNAs were selected (hsa-let-7f-5p (SEQ ID NO: 3) (referred to in the following also as let-7f), hsa-miR-197-3p (SEQ ID NO: 4) (referred to in the following also as miR-197), hsa-miR-223-3p (SEQ ID NO: 5) (referred to in the following also as miR-223), hsa-miR-379-5p (SEQ ID NO: 6) (referred to in the following also as miR-379), and hsa-miR-93-5p (SEQ ID NO: 7) (referred to in the following also as miR-93). These miRNAs were significantly deregulated in all groups of patients with heart diseases (*n*=184) including DCM (*n* = 25) compared to healthy donors (*n* = 25) (cf. Table 3 and Figure 1).

In this respect, Figure 1 shows a volcano plot of comparisons of expression of 323 miRNAs assessed in OpenArray analysis isolated from serum of patients with inflammatory and/or virally induced myocardial diseases (n=184) and DCM (n=25), and healthy controls (n=25). The volcano plot displays the relationship between fold change and significance between the two groups, applying a student's t-test. The y-axis depicts the negative log 10 of p-values of the t-tests (the horizontal slider at 1.3 corresponds to a p-value of 0.05, a higher value indicates greater significance) and the x-axis is the difference in expression between the two experimental groups as log2 fold. Highlighted are five abundant miRNAs (let-7f, miR-197, miR-223, miR-379, and miR-93) significantly expressed in all study groups (cf. also Table 3).

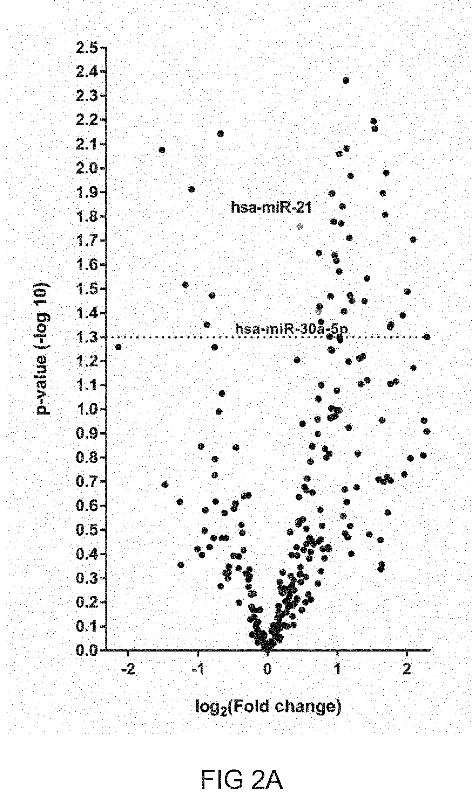

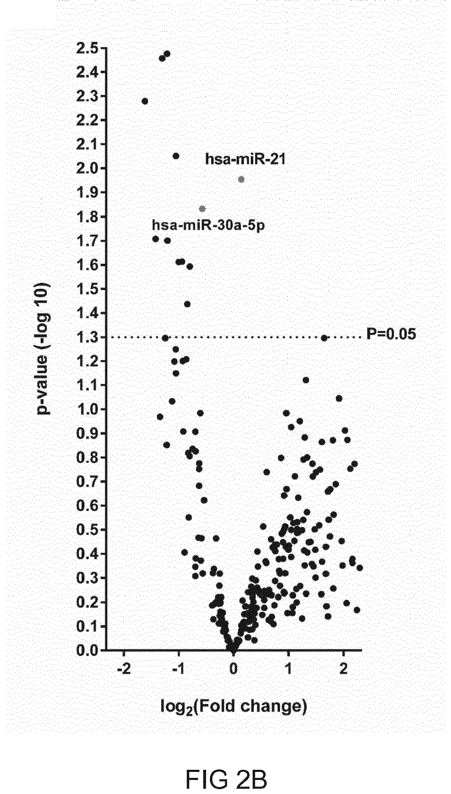

Figure 2A shows a volcano plot of a comparison of 323 miRNAs assessed in OpenArray analysis isolated from serum of DCM patients (n=25) and healthy controls (n=25). Figure 2B shows a volcano plot of a comparison of 323 miRNAs assessed in OpenArray analysis isolated from serum of DCM patients (n=25) and patients with inflammatory and/or virally induced (n=184) myocardial disease. Highlighted are two abundant miRNAs (hsa-miR-21-5p (SEQ ID NO: 1) (referred to in the following also as miR-21) and hsa-miR-30a-5p (SEQ ID NO: 2) (referred to in the following also as miR-30a-5p) significantly expressed in DCM group (cf. also Table 3).

Summarizing, based on the expression levels of miRNAs, miR-21 and miR-30a-5p were significantly deregulated in patients with DCM (*n* = 25) compared to healthy donors (*n* = 25) (Figure 2A) and patients with inflammatory and/or virally-induced cardiomyopathies (n=184) (Figure 2B).

### Confirmation of differentially expressed miRNAs using qRT-PCR

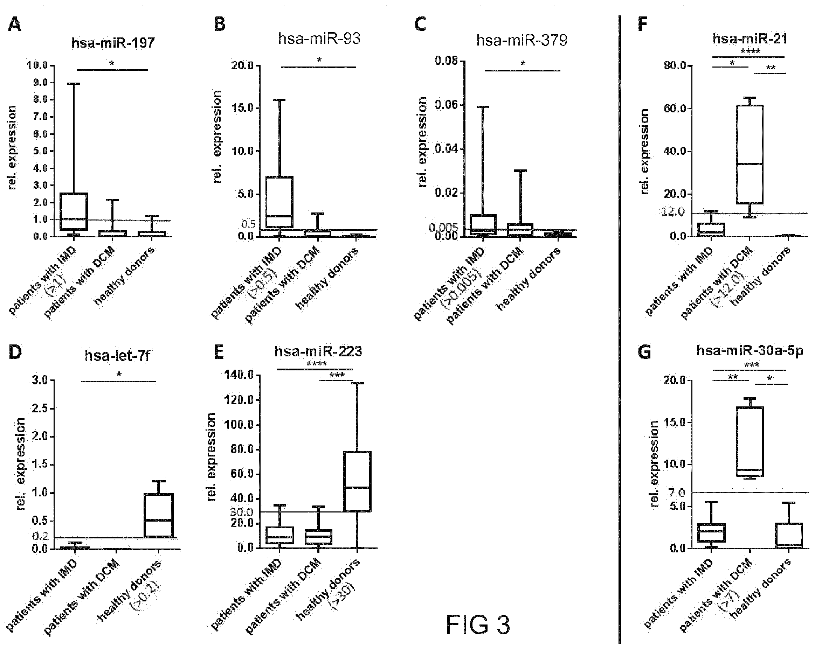

To confirm the significant changes in miRNA expression detected by TaqMan^{®} OpenArray^{®}, these miRNAs were assessed by qRT-PCR using independent sets of another patients with inflammatory and/or virally-induced cardiomyopathies (*n* = 159), DCM patients (*n* = 46) and control healthy subjects (*n* = 60) (Table 4). Let-7f, miR-197, miR-223, miR-379, and miR-93 showed statistically significant deregulation in patients with unexplained heart failure, and miR-21 and miR-30a-5p were significantly deregulated in patients with DCM compared with healthy controls and patients with inflammatory and/or virally-induced cardiomyopathies (Figure 3).

In this respect, Figure 3 also indicates grey lines indicating expression level limits for each miRNA according to single measurements of relative expression normalized to hsa-miR-30a-3p of each patient (* *P* < 0.05, ** *P* <0.005, *** *P* <0.0005, **** *P* <0.0001). These expression level limits or boundaries can be used further to distinguish between healthy donors and patients with IMD (Figure 3, plots A-E) and between healthy donors, patients with IMD, and patients with DCM (Figure 3, plots F and G).

### Setting of expression level limits in three study groups

The achieved results can be used further in diagnostics. Then, it is appropriate to set the expression level limits for each miRNA in three study groups (Table 5). Given their significantly different levels in patient serum, these seven miRNAs (normalized to hsa-miR-30a-3p) appear to have potential as diagnostic biomarkers (cf. Figure 3).

**Table 5: Relative expression level limits in three study groups based on Figure 3. The relative expression of each miRNA is normalized to (referred to) hsa-miR-30a-3p used as housekeeping miRNA.**

| **Diagnosis** | **Relative expression levels** | | | | | | |
|---|---|---|---|---|---|---|---|
| | **hsa-let7f** | **hsa-miR-197** | **mmu-miR-93** | **mmu-miR-379** | **hsa-miR-223** | **hsa-miR-21** | **hsa-miR-30a-5p** |
| **Patients with inflammatory / virally-induced cardiomyopathies** | 0-0,2 | **> 1** | **1 - 10** | **> 0,005** | 0-30 | 0 - 12 | 0-7 |
| **Patients with DCM** | 0-0,2 | 0-1 | 0-3 | 0 - 0,005 | 0-20 | **> 12** | **> 7** |
| **Healthy donors** | **> 0,2** | 0-1 | 0-1 | 0 - 0,005 | **> 30** | 0-1 | 0-1 |

### Diagnostic value of miRNAs for DCM and cardiac diseases

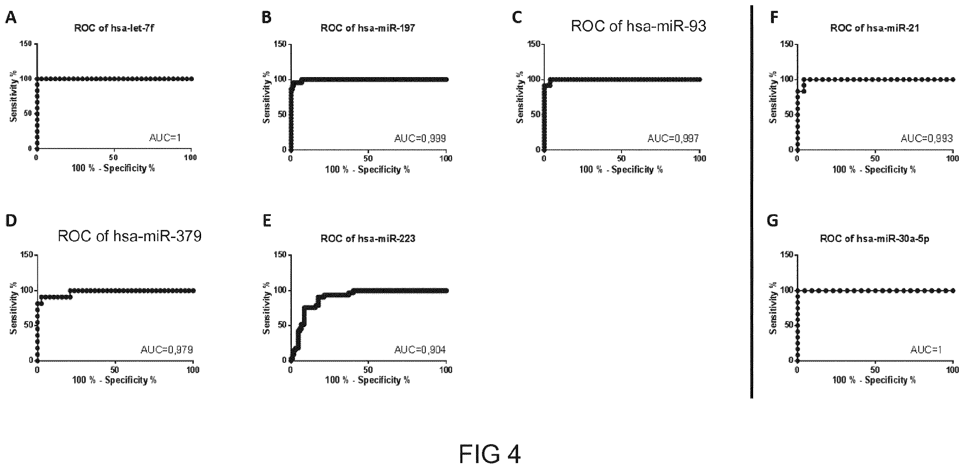

To discriminate between the patients with inflammatory and/or virally-induced cardiomyopathies, DCM patients and healthy controls (diagnostic value), receiver-operating characteristic (ROC) curves were plotted for every single miRNA in confirmation of qRT-PCR that was expressed at a significantly higher level in patient serum (Figure 4). The areas under the curves (AUCs) ranged from 0.904 to 1 in all of miRNAs, proving that these circulating miRNAs are of particular interest for cardiac disease, DCM detection, and diagnosis.

In this respect, Figure 4 shows ROC curves illustrating the diagnostic ability of a binary classifier system. Upon variation the discrimination threshold, the true positive rate (sensitivity) is plotted against the false positive rate (1-specificity). AUC is equal to the probability that a classifier will rank a randomly chosen positive instance higher than a randomly chosen negative one. Plots A to G of Figure 4 correspond to the experiments the results of which are depicted in plots A to G of Figure 3.

### Influence of EF on the miRNA expression

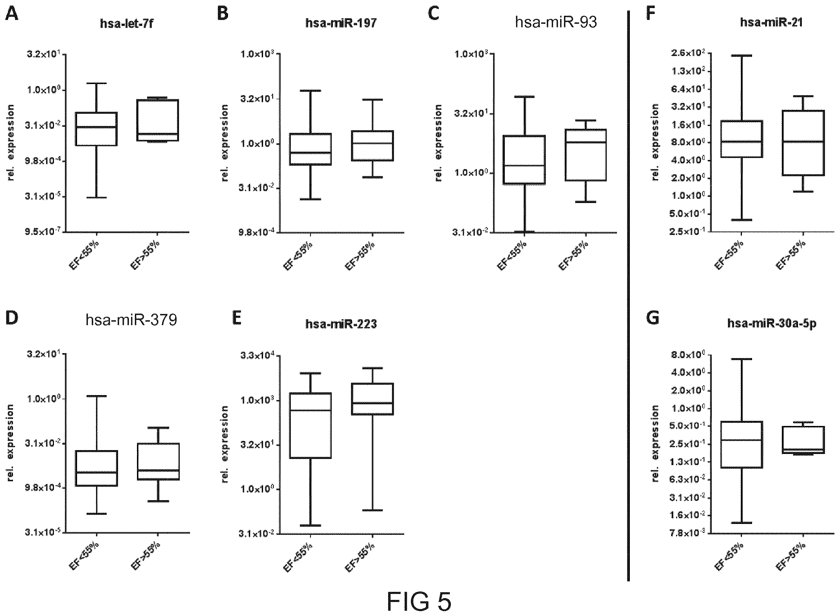

To evaluate the role of miRNAs in the classification of patients with unexplained heart failure and DCM patients, the samples were divided into two subgroups according to ejection fraction (EF) in the conventional transthoracic echocardiography (EF >55%; EF ≤55%) (Figure 5). The relationship between the expression level of circulating miRNAs and cardiac function was further studied. Regarding all selected seven miRNAs, no significant dependence of EF on the miRNA expression level could be detected. This is illustrated in Figure 5. Thus, the selected microRNAs are appropriate biomarkers independent on hemodynamic parameters such as the ejection fraction.

### Discussion

In the exemplary embodiment, a miRNA profile was detected, which matches the criteria for different inflammatory and/or virally-induced cardiomyopathies and DCM. The expression of let-7f, miR-197, miR-223, miR-93, and/or miR-379 allowed to differentiate between patients with a virus or/and inflammation and healthy donors with average specificity of about 93 %.

Based on the expression of miR-21 and/or miR-30a-5p, it was possible to distinguish between patients with DCM, patients with inflammatory and/or virally-induced cardiomyopathies, and healthy controls with a specificity of 95 %. This new approach is of high clinical relevance to clarify the importance of taking an endomyocardial biopsy from patients with suspected inflammation and/or viral heart muscle disease for identification of causative factors, getting a clear diagnosis and biopsy-specific treatment.

Moreover, it was possible to show that EF has no significant influence on the expression level of miRNAs in this setting, meaning that the miRNA profile could recognize patients with inflammatory and/or virally-induced cardiomyopathies and DCM patients only by the expression level of particular miRNAs.

### List of references cited in the preceding sections

1) Wang G-K., Zhu J-Q., Zhang J-T., et al. Circulating microRNA: a novel potential biomarker for early diagnosis of acute myocardial infarction in humans. Eur Heart J 2010;31(6):659-66.
2) Cahill TJ., Kharbanda RK. Heart failure after myocardial infarction in the era of primary percutaneous coronary intervention: Mechanisms, incidence and identification of patients at risk. World J Cardiol 2017;9(5):407-15. Doi: 10.4330/wjc.v9.i5.407.
3) Tijsen AJ., Creemers EE., Moerland PD., et al. MiR423-5p As a Circulating Biomarker for Heart Failure 2010;106(6):1035-9. Doi: 10.1161/circresaha.110.218297.
4) Gidlöf O., Smith JG., Miyazu K., et al. Circulating cardio-enriched microRNAs are associated with long-term prognosis following myocardial infarction. BMC Cardiovasc Disord 2013;13(1):12. Doi: 10.1186/1471-2261-13-12.
5) Williams AH., Liu N., van Rooij E., Olson EN. MicroRNA control of muscle development and disease. Curr Opin Cell Biol 2009;21. Doi: 10.1016/j.ceb.2009.01.029.
6) Zhao Y., Samal E., Srivastava D. Serum response factor regulates a muscle-specific microRNA that targets Hand2 during cardiogenesis. Nature 2005;436. Doi: 10.1038/nature03817.
7) Tian J., An X., Niu L. Role of microRNAs in cardiac development and disease. Exp Ther Med 2017;13(1):3-8. Doi: 10.3892/etm.2016.3932.
8) Schultheiss H-P., Fairweather D., Caforio ALP., et al. Dilated cardiomyopathy. Nat Rev Dis Prim 2019;5(1):32. Doi: 10.1038/s41572-019-0084-1.
9) Cavarretta E., Frati G. MicroRNAs in Coronary Heart Disease: Ready to Enter the Clinical Arena? Biomed Res Int 2016;2016:2150763. Doi: 10.1155/2016/2150763.
10) Pauschinger M., Phan MD., Doerner A., et al. Enteroviral RNA replication in the myocardium of patients with left ventricular dysfunction and clinically suspected myocarditis. Circulation 1999;99(7):889-95.
11) Thomas Aretz H. Myocarditis: The Dallas criteria. Hum Pathol 1987;18(6):619-24. Doi: https://doi.org/10.1016/S0046-8177(87)80363-5.
12) Caforio ALP., Pankuweit S., Arbustini E., et al. Current state of knowledge on aetiology, diagnosis, management, and therapy of myocarditis: a position statement of the European Society of Cardiology Working Group on Myocardial and Pericardial Diseases. Eur Heart J 2013;34(33):2636-48, 2648a-2648d. Doi: 10.1093/eurheartj/eht210.
13) Kühl U., Noutsias M., Schultheiss HP. Immunohistochemistry in dilated cardiomyopathy. Eur Heart J 1995;16 Suppl O:100-6.
14) Escher F., Kühl U., Lassner D., et al. High Perforin-Positive Cardiac Cell Infiltration and Male Sex Predict Adverse Long-Term Mortality in Patients With Inflammatory Cardiomyopathy. J Am Heart Assoc 2017;6(8):e005352. Doi: 10.1161/JAHA.116.005352.
15) Kuhl U., Lassner D., Dorner A., et al. A distinct subgroup of cardiomyopathy patients characterized by transcriptionally active cardiotropic erythrovirus and altered cardiac gene expression. Basic Res Cardiol 2013;108(5):372. Doi: 10.1007/s00395-013-0372-y.
16) Escher F., Tschöepe C., Lassner D., Schultheiss H-P. Myocarditis and inflammatory cardiomyopathy: from diagnosis to treatment. Turk Kardiyol Dern Ars 2015;43(8):739-48.
17) Kühl U., Pauschinger M., Noutsias M., et al. High Prevalence of Viral Genomes and Multiple Viral Infections in the Myocardium of Adults With "Idiopathic" Left Ventricular Dysfunction. Circulation 2005;111(7):887-93. Doi: 10.1161/01.CIR.0000155616.07901.35.
18) Kühl U., Schultheiss H. Viral myocarditis. Swiss Med Wkly 2014;144:w14010. Doi: 10.4414/smw.2014.14010.

## Claims

1. In-vitro method for identifying an early stage of a cardiomyopathy independent of a hemodynamic impairment of a patient's heart,
**characterized**
**in that** a blood serum/blood plasma sample of a patient is analyzed to determine, in a semiquantitative or quantitative manner, an expression level of at least one microRNA selected from the group consisting of the following microRNAs: hsa-miR-21-5p (SEQ ID NO: 1) and hsa-miR-30a-5p (SEQ ID NO: 2), wherein an expression level of the at least one microRNA higher than in a healthy control group and/or a group of patients suffering from an inflammatory myocardial disease and/or a group of patients suffering from a virally-induced cardiomyopathy is taken as indicator for the presence of an early stage of a cardiomyopathy, wherein the early stage of a cardiomyopathy is **characterized by** an absence of a viral infection of the patient's heart and/or an absence of an inflammation of the patient's heart.

2. In-vitro method according to claim 1, **characterized in that** the patient has no clinically noticeable cardiac insufficiency.

3. In-vitro method according to claim 1 or 2, **characterized in that** an expression level of at least one additional microRNA selected from the group consisting of the following microRNAs is determined in a semiquantitative or quantitative manner: hsa-let-7f-5p (SEQ ID NO: 3), hsa-miR-197-3p (SEQ ID NO: 4), hsa-miR-223-3p (SEQ ID NO: 5), hsa-miR-379-5p (SEQ ID NO: 6), and hsa-miR-93-5p (SEQ ID NO: 7).

4. In-vitro method according to any of the preceding claims, **characterized in that** the following steps are carried out:
a) providing a sample obtained from an apparently healthy individual or a patient suspected of suffering from a cardiomyopathy,
b) bringing the sample into contact with at least one probe comprising a sequence corresponding to any sequence of one of the microRNAs from the group as set forth in claim 1 and optionally from the group as set forth in claim 3, or complementary to these, under conditions allowing hybridization between microRNAs contained in the sample and the at least one probe,
c) determining hybridization between microRNAs of the sample and the at least one probe in a semiquantitative or quantitative manner, and
d) confirming a relative or absolute expression level of a microRNA in the sample using the hybridization result of the previous step.

5. Use of at least one microRNA selected from the group consisting of hsa-miR-21-5p (SEQ ID NO: 1) and hsa-miR-30a-5p (SEQ ID NO: 2) as marker in an in-vitro method for identifying an early stage of a cardiomyopathy independent of a hemodynamic impairment of a patient's heart, wherein an expression level of the at least one microRNA is determined in a blood serum/blood plasma sample of a patient in a semiquantitative or quantitative manner.

## Patentansprüche

1. In-vitro-Verfahren zur Identifizierung eines Frühstadiums einer Kardiomyopathie unabhängig von einer hämodynamischen Beeinträchtigung des Herzens eines Patienten,
**dadurch gekennzeichnet**
**dass** eine Blutserum-/Blutplasmaprobe eines Patienten analysiert wird, um auf semiquantitative oder quantitative Weise ein Expressionsniveau von zumindest einer microRNA zu bestimmen, die aus der Gruppe ausgewählt ist, die aus den folgenden microRNAs besteht: hsa-miR-21-5p (SEQ ID NO: 1) und hsa-miR-30a-5p (SED ID NO: 2), wobei ein Expressionsniveau der zumindest einen microRNA, das höher ist als in einer gesunden Kontrollgruppe und/oder einer Gruppe von Patienten mit einer entzündlichen Herzmuskelerkrankung und/oder einer Gruppe von Patienten mit einer virusinduzierten Kardiomyopathie, als Indikator für das Vorliegen eines Frühstadiums einer Kardiomyopathie angesehen wird, wobei das Frühstadium einer Kardiomyopathie durch ein Fehlen einer Virusinfektion des Herzens des Patienten und/oder ein Fehlen einer Entzündung des Herzens des Patienten gekennzeichnet ist.

2. In-vitro-Verfahren nach Anspruch 1, **dadurch gekennzeichnet, dass** der Patient keine klinisch auffällige Herzinsuffizienz hat.

3. In-vitro-Verfahren nach Anspruch 1 oder 2, **dadurch gekennzeichnet, dass** ein Expressionsniveau von zumindest einer zusätzlichen microRNA, ausgewählt aus der Gruppe bestehend aus den folgenden microRNAs, auf semi-quantitative oder quantitative Weise bestimmt wird: hsa-let-7f-5p (SEQ ID NO: 3), hsa-miR-197-3p (SED ID NO: 4), hsa-miR-223-3p (SEQ ID NO: 5), hsa-miR-379-5p (SEQ ID NO: 6), und hsa-miR-93-5p (SEQ ID NO: 7).

4. In-vitro-Verfahren nach einem der vorhergehenden Ansprüche, **dadurch gekennzeichnet, dass** die folgenden Schritte durchgeführt werden:
a) Bereitstellen einer Probe, die von einer offensichtlich gesunden Person oder von einem Patienten mit Verdacht auf Kardiomyopathie entnommen wurde,
b) Inkontaktbringen der Probe mit zumindest einer Sonde, die eine Sequenz umfasst, die einer beliebigen Sequenz einer der microRNAs aus der Gruppe, wie sie nach Anspruch 1 definiert ist, und optional aus der Gruppe, wie sie nach Anspruch 3 definiert ist, entspricht, oder ergänzend unter Bedingungen, die eine Hybridisierung zwischen den in der Probe enthaltenen microRNAs und der zumindest einen Sonde ermöglichen,
c) Bestimmen der Hybridisierung zwischen microRNAs der Probe und der zumindest einen Sonde auf eine semiquantitative oder quantitative Weise, und
d) Bestätigen eines relativen oder absoluten Expressionsniveaus einer microRNA in der Probe unter Verwendung des Hybridisierungsergebnisses des vorherigen Schritts.

5. Verwendung von zumindest einer microRNA, ausgewählt aus der Gruppe bestehend aus hsa-miR-21-5p (SEQ ID NO: 1) und hsa-miR-30a-5p (SEQ I NO: 2) als Marker in einem in vitro-Verfahren zur Identifizierung eines Frühstadiums einer Kardiomyopathie unabhängig von einer hämodynamischen Beeinträchtigung des Herzens eines Patienten, wobei ein Expressionsniveau der zumindest einen microRNA in einer Blutserum-/Blutplasma-Probe eines Patienten auf semi-quantitative oder quantitative Weise bestimmt wird.

## Revendications

1. Procédé in vitro d'identification d'un stade précoce d'une cardiomyopathie indépendamment d'une déficience hémodynamique du coeur d'un patient,
**caractérisé**
**en ce qu'**un échantillon de sérum sanguin/de plasma sanguin d'un patient est analysé pour déterminer, d'une manière semi-quantitative ou quantitative, un niveau d'expression d'au moins un micro-ARN choisi parmi le groupe constitué des micro-ARN suivants : hsa-miR-21-5p (SEQ ID NO : 1) et hsa-miR-30a-5p (SED ID NO : 2), dans lequel un niveau d'expression de l'au moins un micro-ARN supérieur à celui d'un groupe témoin sain et/ou d'un groupe de patients atteints d'un myocardiopathie inflammatoire et/ou d'un groupe de patients atteints d'une cardiomyopathie induite par des virus est considéré comme un indicateur de la présence d'un stade précoce d'une cardiomyopathie, dans lequel le stade précoce d'une cardiomyopathie est **caractérisé par** une absence d'une infection virale du coeur du patient et/ou une absence d'une inflammation du coeur du patient.

2. Procédé in vitro selon la revendication 1, **caractérisé en ce que** le patient ne présente aucune insuffisance cardiaque apparente cliniquement.

3. Procédé in vitro selon la revendication 1 ou 2, **caractérisé en ce qu'**un niveau d'expression d'au moins un micro-ARN supplémentaire choisi parmi le groupe constitué des micro-ARN suivants est déterminé selon une manière semi-quantitative ou quantitative : hsa-let-7f-5p (SEQ ID NO : 3), hsa-miR-197-3p (SED ID NO : 4), hsa-miR-223-3p (SEQ ID NO : 5), hsa-miR-379-5p (SEQ ID NO : 6), et hsa-miR-93-5p (SEQ ID NO : 7).

4. Procédé in vitro selon l'une quelconque des revendications précédentes, **caractérisé en ce que** les étapes suivantes sont réalisées :
a) la fourniture d'un échantillon prélevé chez un individu apparemment sain ou chez un patient soupçonné d'être atteint d'une cardiomyopathie,
b) la mise en contact de l'échantillon avec au moins une sonde comprenant une séquence correspondant à une quelconque séquence d'un des micro-ARN issus du groupe tel que défini selon la revendication 1 et, en option, issus du groupe tel que défini selon la revendication 3, ou, en complément, dans des conditions permettant l'hybridation entre des micro-ARN contenus dans l'échantillon et l'au moins une sonde,
c) la détermination de l'hybridation entre des micro-ARN de l'échantillon et l'au moins une sonde de manière semi-quantitative ou de manière quantitative, et
d) la confirmation d'un niveau d'expression relatif ou absolu d'un micro-ARN dans l'échantillon en utilisant le résultat d'hybridation de l'étape précédente.

5. Utilisation d'au moins un micro-ARN choisi parmi le groupe constitué de hsa-miR-21-5p (SEQ ID NO : 1) et hsa-miR-30a-5p (SEQ I NO : 2) en tant que marqueur dans un procédé in vitro pour identifier un stade précoce d'une cardiomyopathie indépendamment d'une déficience hémodynamique du coeur d'un patient, dans laquelle un niveau d'expression de l'au moins un micro-ARN est déterminé dans un échantillon de sérum sanguin/de plasma sanguin d'un patient d'une manière semi-quantitative ou quantitative.
